# EUROPEAN PATENT SPECIFICATION

(11) **EP 4 288 413 B1**
(45) Date of publication and mention of the grant of the patent: **22.01.2025**
(21) Application number: 21706850.1
(22) Date of filing: 03.02.2021
(51) Int. Cl.: C07C 405/00, C07C 201/02, C07C 203/04

(54) **PROCESS FOR THE PREPARATION OF A NITRIC OXIDE DONATING PROSTAGLANDIN ANALOGUE**
VERFAHREN ZUR HERSTELLUNG EINES STICKOXIDABGEBENDEN PROSTAGLANDINANALOGS
PROCÉDÉ DE PRÉPARATION D'UN ANALOGUE DE PROSTAGLANDINE DONNEUR D'OXYDE NITRIQUE

(43) Date of publication of application: 13.12.2023
(73) Proprietor: Nicox S.A., 06410 Biot (FR)
(72) Inventor: ALMIRANTE, Nicoletta, 20155 MILAN (IT); RONSIN, Gael, 67000 STRASBOURG (FR); DIKER, Khalid, 60740 SAINT MAXIMIN (FR); GORINS, Gilles, 94170 LE PERREUX SUR MARNE (FR); COUSIN, Jonathan, 95260 BEAUMONT SUR OISE (FR); BRUNEAU, Audrey, 60550 VERNEUIL-EN-HALATTE (FR)
(74) Representative: Bianchetti & Minoja with Trevisan & Cuonzo IPS SRL
(86) International application number: PCT/EP2021/052553
(87) International publication number: WO 2022/167070

(56) References cited:
- EP-A1- 3 772 511
- WO-A1-2009/136281
- WO-A1-2019/162149

## Description

### Field of the invention

The present invention relates to a process suitable for large scale preparation of hexanoic acid, 6-(nitrooxy)-, (1S,2E)-3-[(1R,2R,3S,5R)-2-[(2Z)-7-(ethylamino)-7-oxo-2-hepten-1-yl]-3,5-dihydroxycyclopentyl]-1-(2-phenylethyl)-2-propen-1-yl ester of formula (I) that allows to obtain said product having a high chemical purity. The present invention also describes the preparation of highly pure 6-(nitrooxy)hexanoic acid (Villa) that is a key intermediate of the synthesis.

### Background of the invention

Hexanoic acid, 6-(nitrooxy)-, (1S,2E)-3-[(1R,2R,3S,SR)-2-[(2Z)-7-(ethylamino)-7-oxo-2-hepten-1-yl]-3,5-dihydroxycyclopentyl]-1-(2-phenylethyl)-2-propen-1-yl ester of formula (I) 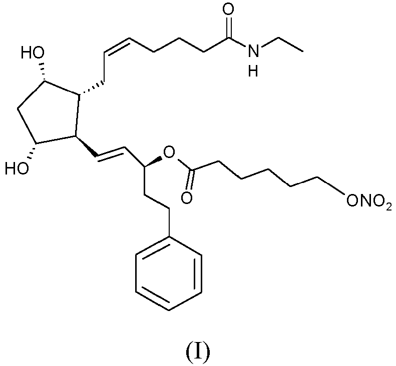 is a prostaglandin analogue that has proved effective as IOP-lowering agent (Impagnatiello F, Toris CB, Batugo M, Prasanna G, Borghi V, Bastia E, Ongini E, Krauss AHP; Invest Ophthalmol Vis Sci. 2015; 56:6558-64).

Two ophthalmic solutions containing compound (I) as active pharmaceutical ingredient (0.065% and 0.1%) are being evaluated for the lowering of intraocular pressure (IOP) in patients with open-angle glaucoma or ocular hypertension in a first Phase 3 clinical trial started on June 2020.

In the past few years, various regulatory authorities have been emphasizing on the purity requirements and the identification of impurities in the Active Pharmaceutical Ingredients (API). Currently, any impurity is considered an organic material, besides the drug substance, that may influence the efficacy and the safety of the pharmaceutical product. Therefore, the identification and the quantification of each impurity, especially of those bearing structural alert for mutagenicity, have become mandatory regulatory requirements. In addition, since the Active Pharmaceutical Ingredients are intended for pharmaceutical use, reagents, solvents, catalysts, etc. which can be used in the synthesis of the active ingredients are limited to those having pharmaceutical industry acceptability.

Hexanoic acid, 6-(nitrooxy)-, (1S,2E)-3-[(1R,2R,3S,5R)-2-[(2Z)-7-(ethylamino)-7-oxo-2-hepten-1-yl]-3,5-dihydroxycyclopentyl]-1-(2-phenylethyl)-2-propen-1-yl ester (I) is an oil and its large-scale purification is difficult because compound (I) cannot be crystallized, therefore the presence of impurities is a critical issue for a large-scale production.

It is known from the prior art processes for the preparation of compound (I) that the main impurities are: the 15-(6-chlorohexanoyl) ester of bimatoprost of formula (V) when the nitration step is performed at the end of the synthesis as disclosed in WO 2009/136281 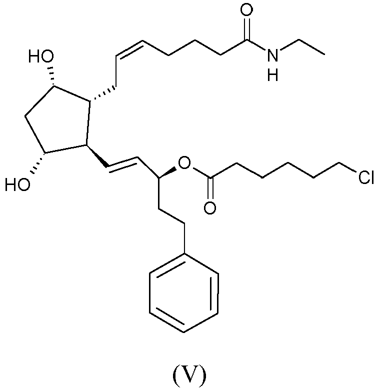 or the side-products of the nitration step of the preparation of the intermediate 6-(nitrooxy)hexanoic acid (Villa) that lead to the formation of the 6-{[6-(nitrooxy)hexanoyl]oxy}hexanoic acid ester of bimatoprost (VII) 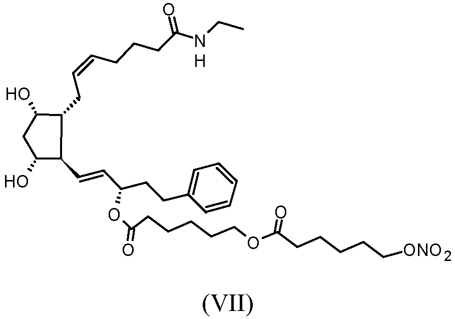 Therefore, the purity of the reagents and the control of the reactions conditions are important requirements for obtaining the compound (I) having a pharmaceutical acceptable purity.

A process for preparing compound of formula (I) is disclosed in WO 2009/136281. WO 2009/136281 discloses the synthesis of compound (I) and in general the preparation of 15-alkyl nitrate esters of bimatoprost.

WO 2009/136281 discloses the synthesis of compound of formula (I) (Example B-1) by reacting bimatoprost in a boronate protected form (II) with 6-bromohexanoyl chloride to give the 15-(6-bromohexanoyl) ester of bimatoprost in a boronate protected form of formula (III) that is converted into the nitrate derivative by silver nitrate in acetonitrile, deprotected and purified under reverse phase chromatography yielding compound of formula (I).

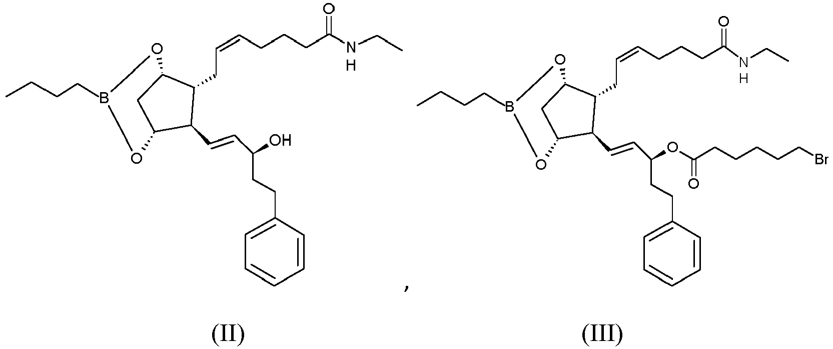

The main disadvantages of the above synthesis are the use in the esterification reaction of more than an equimolar amount of 6-bromohexanoyl chloride, which presents a structural alert for potential mutagenicity, and, in the last step, the use of silver nitrate that generates a large amount of silver salts in wastewater. Another main disadvantage of this process is the formation of impurities and by products such as 15-(6-bromohexanoyl) ester of bimatoprost of formula (IV)) and 15-(6-chlorohexanoyl) ester of bimatoprost of formula (V) that are difficult to be removed even after multiple purifications because they have similar polarity in chromatography, similar lipophilicity and/or solubility as those of compound (I).

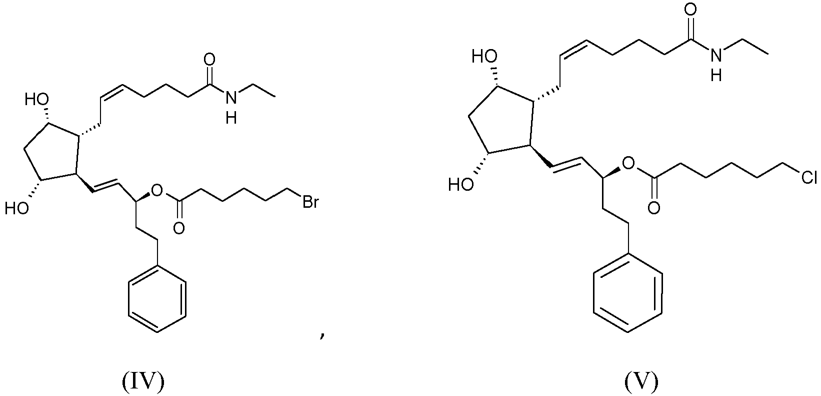

Moreover, compound (V) is predicted as positive for bacterial in vitro mutagenicity based on both a statistical-based method and on an expert rule-based method, as required by regulatory agencies. Compound (IV) also has a halogenated chain recognized as structural alert for potential genotoxicity. Removal of these impurities requires repeated purifications, which further reduces the yield and increases the cost of preparation of compound (I) on a commercial scale.

According to the procedure disclosed in WO 2009/136281, 15-(6-bromohexanoyl) ester of bimatoprost (IV) is an impurity deriving from uncompleted reaction of compound (III) with silver nitrate, after removal of the boronate protection.

The 15-(6-chlorohexanoyl) ester of bimatoprost (V) is a by-product that is formed during the esterification reaction by halogen exchange reaction between the bromine atom of 15-(6-bromohexanoyl) ester of bimatoprost in a boronate protected form (III) and the free chlorine anion of 4-dimethylaminopyridine hydrochloride. The 15-(6-chlorohexanoyl) ester of bimatoprost in a boronate protected form (VI) 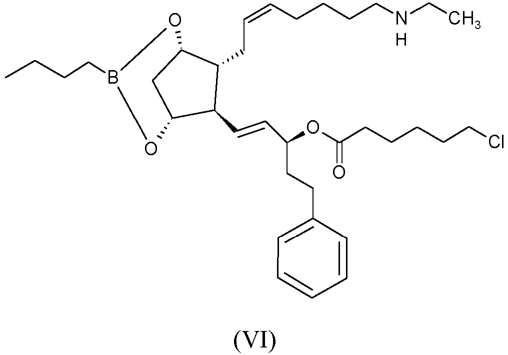 does not react with silver nitrate and, after removal of the protective group, yields compound (V).

WO 2009/136281 also discloses an alternative process for the preparation of 15-acylalkynitrate bimatoprost derivatives (Examples N-1 and O-1). The synthesis comprises reacting bimatoprost in a boronate protected form (II) with a nitrate-alkyl carboxylic acid chloride in the presence of 4-dimethylaminopyridine (DMAP) supported on resin (PS-DMAP), followed by removal of the boronate protecting group and purification using silica gel chromatography.

The above process avoids the use of 6-bromohexanoyl chloride and the removal of the silver salts from the final product. However, this method presents another main disadvantage that is the use of 4-dimethylaminopyridine supported on resin which makes the process unsuitable for commercial scale-up and expensive. Furthermore, nitrate-alkyl carboxylic acid chloride is added in two successive steps and in high excess with respect to compound of formula (II), indeed the alkyl carboxylic acid chloride is added in an amount from about 2 to 4 equivalents.

WO 2009/136281 also discloses another process (Examples Q1) for the preparation of 15-acylalkynitrate bimatoprost derivatives. In this process the compounds were obtained by esterification of bimatoprost in a boronate protected form (II) with an excess of nitrate-alkyl-(p-nitrophenyl)-carboxylate in the presence of 4-dimethylaminopyridine.

The removal of the unreacted nitrate-alkyl-(p-nitrophenyl)-carboxylate and of the by-product p-nitrophenol, which is formed in equimolar amount to compound of formula (I), using chromatographic methods are the main disadvantages of this process.

WO 2016/155906 discloses 15-nitrooxyderivatives of fluprostenol and it reports the synthesis of the 15-nitrooxy-hexyl ester of fluprostenol isopropyl ester. The compound was prepared by reacting fluprostenol isopropyl ester in a boronate protected form with (4-nitrophenyl)-6-nitrooxyhexanoate in the presence of 4-dimethylaminopyridine excess.

As reported above the removal of the unreacted nitrate-alkyl-(p-nitrophenyl)-carboxylate and, especially, the removal of the p-nitrophenol by-product by chromatography methods are the main disadvantages of this process.

WO 2019/162149 discloses a process for the preparation of hexanoic acid, 6-(nitrooxy), (1S,2E)-3-[(1R,2R,3S,SR)-2-[(2Z)-7-(ethylamino)-7-oxo-2-hepten-1-yl]-3,5-dihydroxy cyclopentyl]-1-(2-phenylethyl)-2-propen-1-yl ester (I). The compound (I) is prepared by coupling bimatoprost in a boronate protected form with 6-(nitrooxy)hexanoyl chloride and removing the boronate protecting group. The crude compound (I) is purified by column chromatography.

The 6-(nitrooxy)hexanoyl chloride is synthetized from the 6-(nitrooxy)hexanoic acid that is prepared by a ring-opening reaction of ε-caprolactone and subsequent nitration of the 6-hydroxyhexanoic acid alkali salt with a mixture of HNO₃ and H₂SO₄ in dichloromethane. The 6-(nitrooxy)hexanoic acid is used for the preparation of the correspondent acyl chloride without purification, and also the crude 6-(nitrooxy)hexanoyl chloride is used without purification.

Experiments conducted by the inventors showed that compound (I) prepared using the WO 2019/162149 process contains about 0.1% to 0.4% of the impurity 6-{[6-(nitrooxy)hexanoyl]oxy}hexanoic acid ester of bimatoprost of formula (VII) 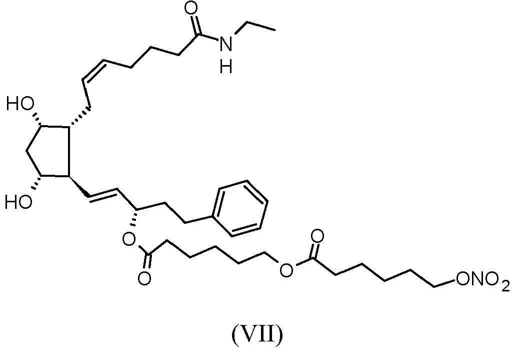 Compounds (I) and (VII) have similar polarity therefore the separation of the two compounds required a cumbersome column purification in order to remove the impurity (VII), therefore the yield of the final product is reduced.

Thus there is a need to develop an industrially viable process for the preparation of high pure hexanoic acid, 6-(nitrooxy)-, (1S,2E)-3-[(1R,2R,3S,5R)-2-[(2Z)-7-(ethylamino)-7-oxo-2-hepten-1-yl]-3,5-dihydroxycyclopentyl]-1-(2-phenylethyl)-2-propen-1-yl ester (I) in high yield.

It was observed that in the WO 2019/162149 process the impurity of formula (VII) is formed in the coupling step because the crude 6-(nitrooxy)hexanoyl chloride of formula (VIIIb) contains the dimer impurity 6-[6-nitrooxyhexanoyl]oxy}hexanoyl chloride of formula (IXb) 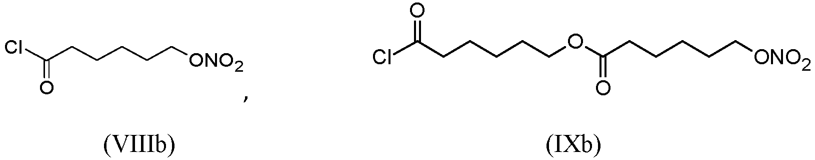 which also reacts with the boronate protected form of bimatoprost (II) and, after removal of the boronate protection, this results in the formation of compound (I) along with the 6-{[6-(nitrooxy)hexanoyl]oxy}hexanoic acid ester of bimatoprost (VII)

The 6-[6-nitrooxyhexanoyl]oxy}hexanoyl chloride of formula (IXb) is a side-product that derives from the crude mixture of the nitration step; indeed the nitration of the 6-hydroxyhexanoic acid alkali metal salt with a mixture of nitric acid and sulfuric acid results in the formation of the 6-(nitrooxy)hexanoic acid (Villa) and the dimer impurity 6-{[6-(nitrooxy)hexanoyl]oxy}hexanoic acid (IXa) 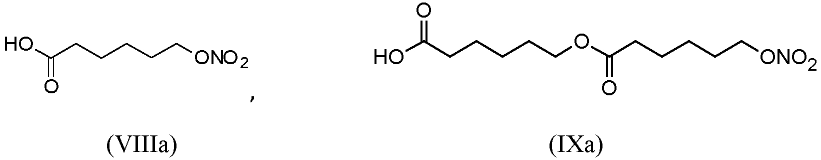 Experiments showed that the nitration mixture contains an amount of compound (Villa) higher than 80% and an amount of compound (IXa) from around 5% to around 9%. Both the compounds (Villa) and (IXa) react with the chlorinating agent leading to a mixture that contains 6-(nitrooxy)hexanoyl chloride (VIIIb) and the dimer impurity 6-[6-nitrooxyhexanoyl]oxy}hexanoyl chloride (IXb). According to the WO 2019/162149 process this crude mixture is reacted with compound (II) without further purification.

In order to control the formation of the impurity (VII), it was surprisingly found that the addition of water, instead of a saturated solution of NaCl in water (brine) as described in WO 2019/162149, during the quenching of the nitration reaction of the 6-hydroxyhexanoic acid alkali salt led to a crude nitration mixture containing a reduced amount of the dimer impurity 6-{[6-(nitrooxy)hexanoyl]oxy}hexanoic acid (IXa). In particular this modification of the work-up of the nitration step yielded a crude nitration mixture containing an amount of 6-(nitrooxy)hexanoic acid (Villa) of 99.2% (a/a %) and an amount of the dimer impurity 6-{[6-(nitrooxy)hexanoyl]oxy}hexanoic acid (IXa) of 0.15% (a/a %). Compounds (Villa) and (IXa) were identified and quantified by appropriate UHPLC analysis.

It is believed that the addition of water at the end of the nitration reaction allows a fast dissolution of the inorganic salts and a better separation of organic phase containing the 6-(nitrooxy)hexanoic acid (Villa) from the acidic aqueous phase containing the unreacted HNO₃ / H₂SO₄, with respect to the addition of a commonly used saturated solution of NaCl in water (brine). This improved work-up of the nitration reaction results in a reduction/inhibition of the side-reactions that lead to the formation of the side-product 6-{[6-(nitrooxy)hexanoyl]oxy}hexanoic acid (IXa).

Furthermore, another advantage of this modification of the nitration step is the reduction of the volume of the organic solvent used for the work-up of the reaction that allows to scale up this step of at least three times fold by.

Therefore, the present invention provides an improved process suitable for large scale production of hexanoic acid, 6-(nitrooxy)-, (1S,2E)-3-[(1R,2R,3S,SR)-2-[(2Z)-7-(ethylamino)-7-oxo-2-hepten-1-yl]-3,5-dihydroxycyclopentyl]-1-(2-phenylethyl)-2-propen-1-yl ester (I) that contains less than 0.05% of the impurity 6-{[6-(nitrooxy)hexanoyl]oxy}hexanoic acid ester of bimatoprost (VII) (measured as a/a% by HPLC.

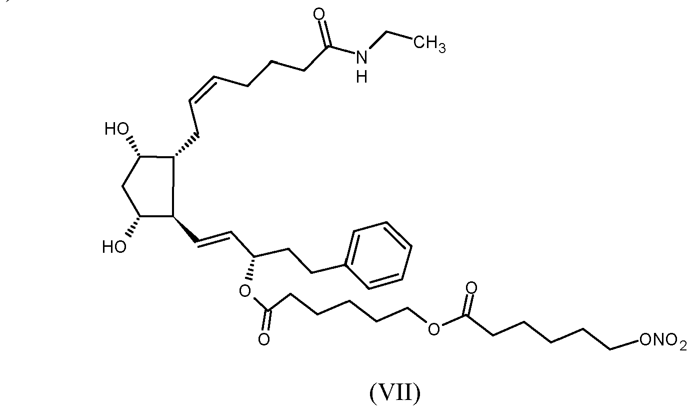

An important advantage of the process of the present invention is that this process is very efficient in the reduction of the level of the impurity (VII) and, at the same time, it provides compound (I) in a good yield. This process is an advantageous alternative to other processes that, although they could theoretically reach the same reduction of the level of the impurity (VII), need to include an additional purification step of the 6-(nitrooxy)hexanoic acid intermediate.

Since the 6-(nitrooxy)hexanoic acid is a liquid and both the 6-(nitrooxy)hexanoic acid (Villa) and the 6-{[6-(nitrooxy)hexanoyl]oxy}hexanoic acid (IXa) are polar compounds, the purification of a large amount of 6-(nitrooxy)hexanoic acid (VIIIa) cannot be easily carried out for industrial scale preparation.

Furthermore, the present invention relates to a process for industrial synthesis of the 6-(nitrooxy)hexanoic acid (Villa) 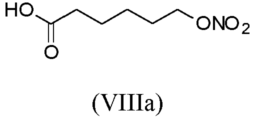 having a purity equal to or greater than 99% and a content of 6-{[6-(nitrooxy)hexanoyl]oxy}hexanoic acid (IXa) equal to or less than 0.2%.

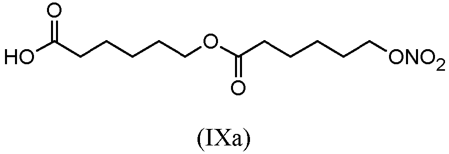

### Description of the invention

The present invention relates to a process for the preparation of hexanoic acid, 6-(nitrooxy)-, (1S,2E)-3-[(1R,2R,3S,5R)-2-[(2Z)-7-(ethylamino)-7-oxo-2-hepten-1-yl]-3,5-dihydroxycyclopentyl]-1-(2-phenylethyl)-2-propen-1-yl ester of formula (I): 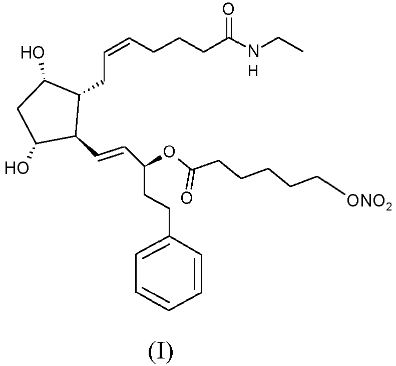 comprising the following steps:
Step A) preparation of the 6-(nitrooxy)hexanoyl chloride (VIIIb) by:
   1a) reacting the ε-caprolactone 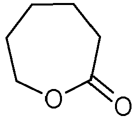 with an inorganic base selected from KOH, NaOH and LiOH in a solvent selected from methanol, ethanol or isopropanol, at a temperature between 20°C and the reflux temperature of the solvent to obtain 6-hydroxyhexanoic acid salt of formula (X) 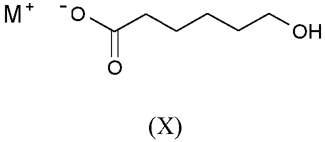 wherein M is K, Na or Li;
   2a) purifying the 6-hydroxyhexanoic acid salt of formula (X) obtained in step 1a) which comprises:
      i. adding methyl tert-butyl ether to the reaction mixture of step 1a) in a ratio methyl tert-butyl ether / volume of the reaction mixture 2:1;
      ii. filtering the solid;
      iii. slurring the solid with a mixture of methyl tert-butyl ether, methanol in a ratio of 3:1 to 5:1 and water in an amount of 0.3 - 1 mole of water for mole of ε-caprolactone;
      iv. isolating the 6-hydroxyhexanoic acid salt of formula (X) as a solid;
   3a) reacting the 6-hydroxyhexanoic acid salt with a mixture of fuming HNO₃ and concentrated H₂SO₄ in dichloromethane, at a temperature ranging from 0°C to 10 °C;
   4a) work-up of the reaction of step 3 a) by adding water to the nitration mixture of step 3a) while keeping the temperature between 0°C and 5 °C;
   5a) separating the organic phase, drying on sodium sulfate said organic phase and distilling off the solvent to obtain crude 6-(nitrooxy)hexanoic acid (Villa) 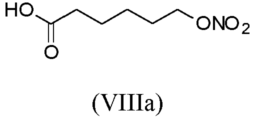 6a) reacting the crude 6-(nitrooxy)hexanoic acid (Villa) with a chlorinating agent in dichloromethane to obtain the crude 6-(nitrooxy)hexanoyl chloride (VIIIb);
Step B) preparation of hexanoic acid, 6-(nitrooxy)-, (1S,2E)-3-[(1R,2R,3S,SR)-2-[(2Z)-7-(ethylamino)-7-oxo-2-hepten-1-yl]-3,5-dihydroxycyclopentyl]-1-(2-phenylethyl)-2-propen-1-yl ester (I) by:
   1b) reacting bimatoprost with butyl boronic acid in methyl tert-butyl ether at temperature about 40°C, to obtain compound (II): 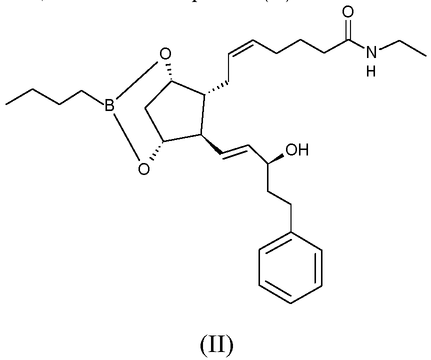
   2b) reacting compound (II) with the crude 6-(nitrooxy)hexanoyl chloride (VIIIb) of step 6a) in the presence of 4-dimethylaminopyridine in free form in an aprotic organic solvent, to obtain the compound (XI) 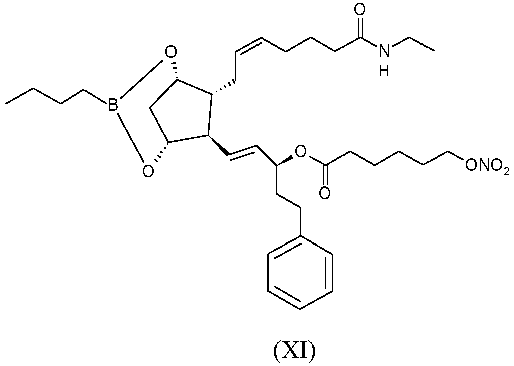
   3b) removing the boronate protective group to obtain the crude compound of formula (I);
   4b) purifying the crude compound (I) by column chromatography;
wherein said process is characterized in that the crude nitration mixture of step 5a) contains an amount equal to or greater than 99% of 6-(nitrooxy)hexanoic acid (VIIIa) and an amount equal to or less than 0.2% of 6-{[6-(nitrooxy)hexanoyl]oxy}hexanoic acid (IXa).

The hexanoic acid, 6-(nitrooxy)-, (1S,2E)-3-[(1R,2R,3S,5R)-2-[(2Z)-7-(ethylamino)-7-oxo-2-hepten-1-yl]-3,5-dihydroxycyclopentyl]-1-(2-phenylethyl)-2-propen-1-yl ester (I) prepared according to the process of the invention has a chemical purity ≥99% and contains less than 0.05% of the impurity 6-{[6-(nitrooxy)hexanoyl]oxy}hexanoic acid ester of bimatoprost (VII). Said compound contains about 0.11% of the impurity 15-(6-chlorohexanoyl) ester of bimatoprost (V). The compounds were identified and quantified by HPLC analysis, the amount of the compounds were expressed as area percentage (a/a%); 0.05% is the HPLC detection limit.

In step 1a) the ring opening reaction of ε-caprolactone is preferably carried out in presence of potassium hydroxide in methanol at temperature between 20 °C and the reflux temperature. During the workup of the reaction mixture of step 1a), the purification of 6-hydroxyhexanoic acid potassium salt is obtained by slurring the solid of step 2a)-ii. in a mixture containing methyl tert-butyl ether, methanol in a ratio of 3:1 to 5:1 and water 0.3 - 1 mole of water for mole of ε-caprolactone (step 2a)-iii.). 6-hydroxyhexanoic acid potassium salt is obtained as a solid.

The crude nitration mixture of step 5a) contains an amount equal to or greater than 99% of 6-(nitrooxy)hexanoic acid (Villa) and an amount of 6-{[6-(nitrooxy)hexanoyl]oxy}hexanoic acid (IXa) equal to or less than 0.2%. The chemical yield of the 6-(nitrooxy)hexanoic acid (Villa) is 80-85%. The chemical purity was assessed by analytical UHPLC method.

The preferred chlorinating agent used in step 6a) is oxalyl chloride.

The step 2b) is carried out at a temperature ranging from 0°C to 25°C and it is preferably carried out in aprotic organic solvent selected from methyl tert-butyl ether, N,N-dimethylformamide or dichloromethane; most preferably, the organic solvent is methyl tert-butyl ether. 4-Dimethylaminopyridine (DMAP) in "free form" means that DMAP is not bound to a resin. The molar ratio of compound (II) to 6-(nitrooxy)hexanoyl chloride (VIIIb) preferably ranges from 1: 1.4 to 1: 1.6.

The molar ratio of compound (II) to 4-dimethylaminopyridine is preferably from 1:2.0 to 1: 2.4.

In step 3b), the removal of the boronate protecting group is preferably carried out using methanol at a temperature from 17°C to 25°C.

A preferred process for preparing the compound of formula (I) comprises the following steps, which are depicted in Scheme 1:

### Step A

1a) reacting the ε-caprolactone with potassium hydroxide in methanol at a temperature between 20°C and reflux temperature of methanol, preferably at reflux temperature of methanol, and
2a) purifying the 6-hydroxyhexanoic acid potassium salt obtained in step 1a) which comprises:
   i. adding methyl tert-butyl ether (MTBE) to the reaction mixture of step 1a) in a ratio of 2:1 MTBE / methanol;
   ii. filtering the solid;
   iii. slurring the solid in a mixture of methyl tert-butyl ether, methanol in a ratio of 3:1 to 5:1 and water in an amount of 0.3 - 1 mole of water for mole of ε-caprolactone;
   iv. isolating the 6-hydroxyhexanoic acid potassium salt as a solid.
3a) adding the 6-hydroxyhexanoic acid potassium salt (formula (X), M = K) to a mixture of fuming HNO₃ and concentrated H₂SO₄ in dichloromethane at a temperature from about 0° C to 10°C and vigorously stirring the reaction mixture at a temperature from 0°C to 5°C and monitoring the progress of the reaction (up to 99.9% of conversion);
4a) carefully adding water to the nitration mixture by and keeping the temperature at 0° to 5°C.
5a) separating the organic phase, drying on sodium sulfate said organic phase and distilling off the solvent to yield crude 6-(nitrooxy)hexanoic acid (Villa) containing an amount of 6-(nitrooxy)hexanoic acid (Villa) equal to or greater than 99% of and an amount of the by-product 6-{[6-(nitrooxy)hexanoyl]oxy}hexanoic acid (IXa) ) equal to less than 0.2%.
6a) reacting the crude 6-(nitrooxy)hexanoic acid with oxalyl chloride to obtain 6-(nitrooxy)hexanoyl chloride (VIIIb) that is used without further purification in Step B of the process;

### Step B

1b) reacting bimatoprost with butyl boronic acid (1.1-1.8 eq) in methyl tert-butyl ether (MTBE) at temperature about 40°C, then removing water by azeotropic distillation to obtain bimatoprost boronate (II);
2b) reacting bimatoprost boronate (II) with the crude 6-(nitrooxy)hexanoyl chloride (VIIIb) obtained in step 6a) (1.4-1.6 equivalent), in methyl tert-butyl ether in the presence of 4-dimethylaminopyridine (2.0-2.4 equivalent) at a temperature ranging from 0°C to 20±3°C, to obtain (1S,2E)-3-{(6R,7R)-3-butyl-7[(2Z)-7-(ethylamino)-7-oxohept-2-en-1-yl]-2,4-dioxa-3-borabicyclo[3.2.1]oct-6-yl}-1-(2-phenylethyl)-prop-2-en-1-yl 6-(nitrooxy)hexanoate (XI);
3b) reacting (1S,2E)-3-{(6R,7R)-3-butyl-7[(2Z)-7-(ethylamino)-7-oxohept-2-en-1-yl]-2,4-dioxa-3-borabicyclo[3.2.1]oct-6-yl}-1-(2-phenylethyl)-prop-2-en-1-yl 6-(nitrooxy) hexanoate (XI) with methanol at room temperature to remove the protective group and to obtain the crude hexanoic acid, 6-(nitrooxy)-, (1S,2E)-3-[(1R,2R,3S,SR)-2-[(2Z)-7-(ethylamino)-7-oxo-2-hepten-1-yl]-3,5-dihydroxycyclopentyl]-1-(2-phenylethyl)-2-propen-1-yl ester (I).
4b) purifying the crude compound (I) by high performance silica gel chromatography to obtain hexanoic acid, 6-(nitrooxy)-,(1S,2E)-3-[(1R,2R,3S,SR)-2-[(2Z)-7-(ethylamino)-7-oxo-2-hepten-1-yl]-3,5-dihydroxycyclopentyl]-1-(2-phenylethyl)-2-propen-1-yl ester (I) using methylene chloride/ methanol as mobile phase

The HPLC quantitative analysis of the isolated compound (I) after chromatography purification had a chemical purity greater than 99% containing an amount of 15-(6-chlorohexanoyl) ester of bimatoprost (V) about 0.11% and an amount of 6-{[6-(nitrooxy)hexanoyl]oxy}hexanoic acid ester of bimatoprost (VII) below the HPLC detection limit of 0.05%.

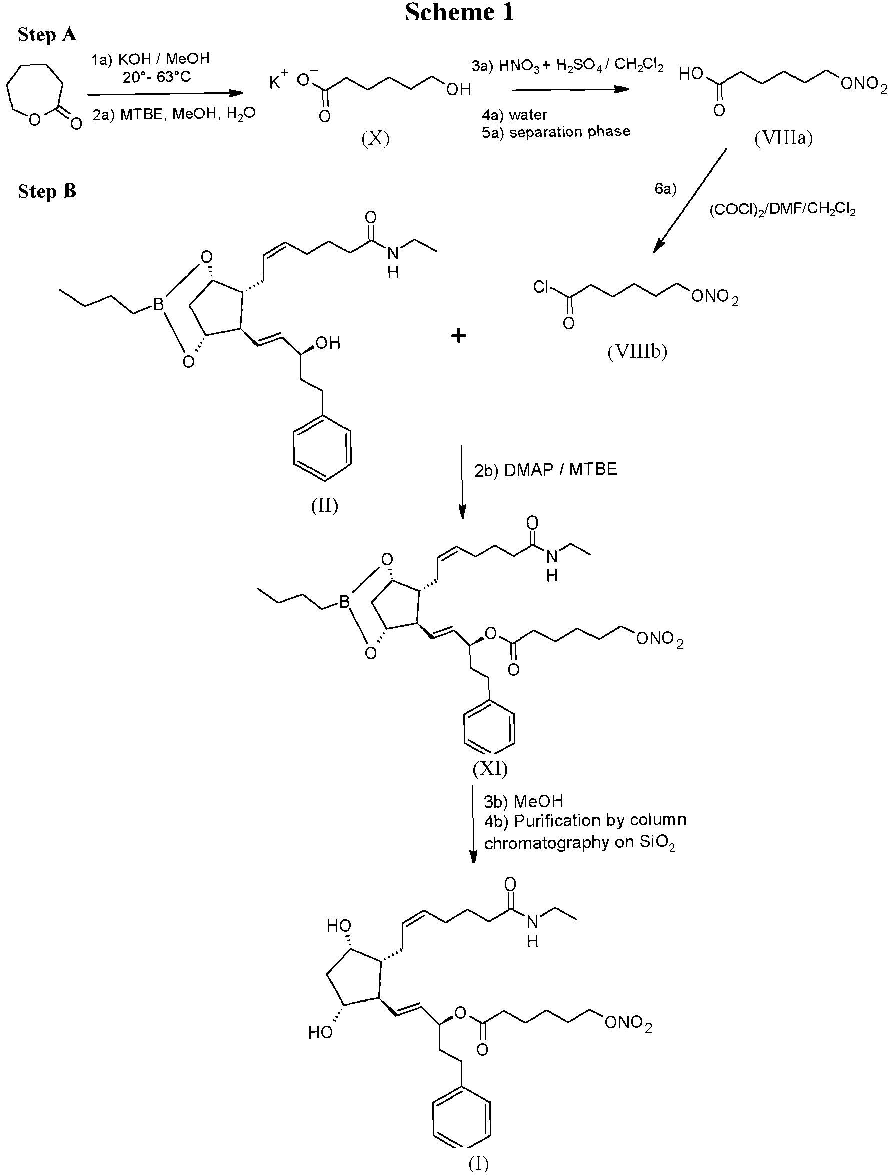

Also disclosed is a pharmaceutical formulation containing hexanoic acid, 6-(nitrooxy)-, (1S,2E)-3-[(1R,2R,3S,SR)-2-[(2Z)-7-(ethylamino)-7-oxo-2-hepten-1-yl]-3,5-dihydroxycyclopentyl]-1-(2-phenylethyl)-2-propen-1-yl ester of formula (I) and at least a pharmaceutically acceptable excipient, wherein hexanoic acid, 6-(nitrooxy)-, (1S,2E)-3-[(1R,2R,3S,5R)-2-[(2Z)-7-(ethylamino)-7-oxo-2-hepten-1-yl]-3,5-dihydroxycyclopentyl]-1-(2-phenylethyl)-2-propen-1-yl ester has a chemical purity of 99%, and contains an amount of 15-(6-chlorohexanoyl) ester of bimatoprost (compound (V)) about 0.11% and an amount of the dimeric impurity 6-{[6-(nitrooxy)hexanoyl]oxy}hexanoic acid ester of bimatoprost (compound (VII)) below 0.05%.

In the present application the quantification of the compounds expressed as percentage % is determined by analysis HPLC (a/a %).

### Examples

All synthesis steps described below were conducted under nitrogen atmosphere.

### Example 1

### Synthesis of hexanoic acid, 6-(nitrooxy)-, (1S,2E)-3-[(1R,2R,3S,SR)-2-[(2Z)-7-(ethylamino)-7-oxo-2-hepten-1-yl]-3,5-dihydroxycyclopentyl]-1-(2-phenylethyl)-2-propen-1-yl ester (compound (I))

### Synthesis of 6-(nitrooxy)hexanoyl chloride (compound (VIIIb))

### Steps 1a) and 2a): Synthesis of 6-hydroxyhexanoic acid potassium salt (compound (X))

A solution of potassium hydroxide (141.12 g corresponding to 122.8g as pure material) in methanol (1250 ml) was prepared under cooling at 15°C to 20°C and was added at 5°C to 37°C within 0.25 hour to a solution of 250.0 g of ε-caprolactone in methanol (675 ml). The mixture was stirred 18 hours at 20°C to 63°C. Then the solvent was concentrated to a volume of approximatively 675 mL and methyl tert-butyl ether (1250 ml) was added at around 57°C. The reaction was stirred for 48 hours at RT and the solid was isolated by filtration to give 380.9 g of crude 6-hydroxyhexanoic acid potassium salt. The crude was re-slurred in MTBE (1475 ml) and Methanol (375 ml) and water (19.7 ml) at 22±3°C for 8 hours and filtered. The solid was re slurred in methyl tert-butyl ether (500 ml) and filtered again and then dried under vacuum at 50°C for 12 hours to give 354.7 g of 6-hydroxyhexanoic acid potassium salt (93.2% yield) with a purity of 98%. Melting point 208°C.

### Steps 3a) to 5a): Synthesis of 6-(nitrooxy)hexanoic acid (compound (Villa))

Fuming HNO₃ (485.5 g, 4.6 equivalents) was added to concentrated H₂SO₄ (553.0 g, 3.1 equivalent) at a temperature from 0°C to 5°C in 30 min, then Dichloromethane (5820 ml) was added at a temperature from 0°C to 5°C in 23 min. 6-Hydroxyhexanoic acid potassium salt (306.09 g, 1 equivalent) was added portion-wise in 35 min at temperature below 5°C. The mixture was stirred for 2 hours at 0°C to 5°C and the reaction was monitored by ¹H-NMR showing 99.9% conversion. Water (2.9 L) was added maintaining temperature between 0°C to 5°C within 20 min. The organic layer was decanted, dried over sodium sulfate and concentrated under vacuum to give 318.4 g of 6-(nitrooxy)hexanoic acid (94.8% yield) having a 99.2% HPLC purity and a content of 6-{[6-(nitrooxy)hexanoyl]oxy}hexanoic acid (compound (IXa)) of 0.15%.

### Step 6a): synthesis of 6-(nitrooxy)hexanoyl chloride (compound (VIIIb))

6-(nitrooxy)hexanoic acid (270.8 g, l equivalent) was dissolved in dichloromethane (1220 ml) and cooled to 0°C to 5°C under nitrogen. Then N,N-dimethylformamide (1.6 ml) and oxalyl chloride (198.2 g) were added at 0°C to 5°C within 30 minutes. The reaction mixture was stirred at 0°C to 5°C for 1 hour and then for 4 hours at 15°C to 20°C. Then the reaction mixture was concentrated under vacuum at temperature equal to or below 40°C and co-evaporated with dichloromethane to give 6-(nitrooxy)hexanoyl chloride (323.9 g, yield 99.6% yield, titration AgNO₃ = 92.0%).

### Step 1b): Synthesis of (Z)-7-[(1S,5R,6R,7R)-3-butyl-6-[((E,3S)-3-hydroxy-5-phenyl-pent-1-enyl]-2,4-dioxa-3-borabicyclo[3.2.1)octan-7-yl]-N-ethyl-hept-5-enamide (compound (II))

Bimatoprost (249.0 g, 1 equivalent) was suspended in Methyl tert-butyl ether (4 L) and Butyl boronic acid (69.0 g, 1.13 equivalents) was added. The mixture was heated to 40°C for 1 hour. The reaction was monitored by ¹H NMR till conversion >97%.

The reaction mixture was cooled down to about 20°C, filtered and rinsed with methyl tert-butyl ether (250 ml). The mixture was concentrated at a temperature about 40°C under vacuum by azeotropic distillation. Rinsing with methyl tert-butyl ether and azeotropic distillation were continued till the water content of (Z)-7-[(1S,5R,6R,7R)-3-butyl-6-[((E,3S)-3-hydroxy-5-phenyl-pent-1-enyl]-2,4-dioxa-3-borabicyclo[3.2.1)octan-7-yl]-N-ethyl-hept-5-enamide (compound (II)) was equal to or below 0.25%.

(Z)-7-[(1S,5R,6R,7R)-3-butyl-6-[((E,3S)-3-hydroxy-5-phenyl-pent-1-enyl]-2,4-dioxa-3-borabicyclo[3.2.1)octan-7-yl]-N-ethyl-hept-5-enamide was isolated as a crude with quantitative yield (313.1 g corresponding to 298.6 g of pure material).

### Step 2b): synthesis of (1S,2E)-3-((6R,7R)-3-butyl-7[(2Z)-7-(ethylamino)-7-oxohept-2-en-1-yl]-2,4-dioxa-3-borabicyclo[3.2.1]oct-6-yl}-1-(2-phenylethyl)-prop-2-en-1-yl 6-(nitrooxy) hexanoate (compound (XI))

(Z)-7-[(1S,5R,6R,7R)-3-Butyl-6-[((E,3S)-3-hydroxy-5-phenyl-pent-1-enyl]-2,4-dioxa-3-borabicyclo[3.2.1)octan-7-yl]-N-ethyl-hept-5-enamide (compound (II)) (313.1 g, 1 equivalent) was dissolved in methyl tert-butyl ether (4.27 L) under nitrogen and cooled to 0°C to 5°C. 4-Dimethylaminopyridine (162.7 g, 2.27 equivalents) was added in one portion. A solution of 6-(nitrooxy)hexanoyl chloride (compound (VIIIb)) (172.1 g, 1.5 equivalents) in methyl tert-butyl ether (600 mL), was added dropwise at 0°C to 5°C within 40 min. After stirring for 2h and 30min at 0°C to 5°C, and at 15° to 20°C for 16.5 hours, the reaction mixture was cooled at 0°C to 5°C and deionized water was added within 20 minutes at a maximum temperature of 10°C.

The mixture was stirred for 5 min. The aqueous layer was separated, and discarded. The organic layer was washed with a 1N hydrochloric acid solution then with deionized water and last with brine.

The organic layer was dried over sodium sulfate and concentrated under vacuum to afford (1S,2E)-3-{(6R,7R)-3-butyl-7[(2Z)-7-(ethylamino)-7-oxohept-2-en-1-yl]-2,4-dioxa -3-borabicyclo[3.2.1]oct-6-yl}-1-(2-phenylethyl)-prop-2-en-1-yl 6-(nitrooxy) hexanoate (compound (XI)) (402.9 g, 94.9% yield)

### Steps 3b) and 4b): Synthesis of hexanoic acid, 6-(nitrooxy)-, (1S,2E)-3-[(1R,2R,3,5R)-2-[(2Z)-7-(ethylamino)-7-oxo-2-hepten-1-yl]-3,5-dihydroxycyclopentyl]-1-(2-phenylethyl)-2-propen-1-yl ester (compound (I))

(1S,2E)-3-{(6R,7R)-3-butyl-7[(2Z)-7-(ethylamino)-7-oxohept-2-en-1-yl]-2,4-dioxa-3-borabicyclo[3.2.1]oct-6-yl}-1-(2-phenylethyl)-prop-2-en-1-yl 6-(nitrooxy) hexanoate (compound (XI)) (396 g crude, (equivalent) was dissolved in methanol (4.20 L) and the resulting solution was stirred at room temperature for 24 hours monitoring the reaction by ¹H-NMR. Then methanol was removed under vacuum at 35°C to 40°C. The residue was dissolved in methanol, stirred for 14 hours and evaporated under vacuum at 35°C to 40°C. The residue was dissolved in methyl tert-butyl ether and washed with deionized water and then brine. The organic layer was dried over sodium sulfate and concentrated under vacuum at a temperature below 40°C to afford the crude hexanoic acid, 6-(nitrooxy)-, (1S,2E)-3-[(1R,2R,3S,SR)-2-[(2Z)-7-(ethylamino)-7-oxo-2-hepten-1-yl]-3,5-dihydroxycyclopentyl]-1-(2-phenylethyl)-2-propen-1-yl ester (352.3 g, 96.5% yield).

The residue was divided in 3 equal amounts (around 107 g each) and purified by chromatography on column using high performance silica gel (SNAP Ultra column, 1500 g of silica) using dichloromethane / methanol as eluent with a gradient 100:0 to 95:5 v/v on a Biotage Isolera LS system. The fractions were monitored by TLC and UHPLC. Fractions were concentrated under vacuum at temperature equal to or below 50°C to give compound (I) as an oil (242.5 g, 90.3% yield).

Hexanoic acid, 6-(nitrooxy)-, (1S,2E)-3-[(1R,2R,3S,SR)-2-[(2Z)-7-(ethylamino)-7-oxo-2-hepten-1-yl]-3,5-dihydroxycyclopentyl]-1-(2-phenylethyl)-2-propen-1-yl ester (Compound (I)) was dissolved in ethanol and treated with charcoal for 30 min, filtered and the solvent was evaporated at a temperature below 50°C under vacuum. Compound (I) was isolated as an oil (212.7 g, 92.4 % yield). The purity of hexanoic acid, 6-(nitrooxy)-, (1S,2E)-3-[(1R,2R,3S,SR)-2-[(2Z)-7-(ethylamino)-7-oxo-2-hepten-1-yl]-3,5-dihydroxycyclopentyl]-1-(2-phenylethyl)-2-propen-1-yl ester (Compound (I)) was of 99.89% (assessed by UHPLC), the content of 15-(6-chlorohexanoyl) ester of bimatoprost (compound (V)) was 0.11% and the content of the dimeric impurity 6-{[6-(nitrooxy)hexanoyl]oxy}hexanoic acid ester of bimatoprost (compound (VII)) was below 0.05%.

### Example 2 (comparative example)

The synthesis reported below was performed according to the method disclosed in WO 2019/162149

### Synthesis of hexanoic acid, 6-(nitrooxy)-, (1S,2E)-3-[(1R,2R,3S,SR)-2-[(2Z)-7-(ethylamino)-7-oxo-2-hepten-1-yl]-3,5-dihydroxycyclopentyl]-1-(2-phenylethyl)-2-propen-1-yl ester (I)

### Synthesis of 6-(nitrooxy)hexanoyl chloride (VIIIb)

### Synthesis of 6-hydroxyhexanoic acid potassium salt (compound (X))

A solution of potassium hydroxide 90% (89.7 g) in methanol (830 ml) was prepared under cooling at 10°C-20°C. 165.2 g of ε-caprolactone (1 eq.) and methanol (415 ml) were introduced in a 4L three-necked round-bottomed flask. The mixture was stirred until dissolution. Then potassium hydroxide solution in methanol was added at 0°-30°C within 25 min. The mixture was stirred for 20 hours at 15°-20°C. The reaction mixture was concentrated under vacuum (at a temperature equal to or below 40°C) to give crude 6-hydroxyhexanoic acid potassium salt. The crude solid was suspended in methyl tert-butyl ether (830 ml) for 2 hours at 15°-20°C, filtered through a pore 3 filter, washed with methyl tert-butyl ether (2×165 ml) and dried under vacuum at 35°C to give 6-hydroxyhexanoic acid potassium salt (228.1g, 98.3% HCl assay) in 92.7% yield as a white powder.

### Synthesis of 6-(nitrooxy)hexanoic acid (VIIIa)

The nitration reaction was performed on around 100g scale in order to control the reaction temperature and to reduce the time of the addition of nitrating mixture. The reaction was performed twice. The nitration mixture was quenched by adding a saturated sodium chloride aqueous solution (brine). Results of the two separate nitration reactions are reported in Table 1.

Nitration 1: 6 L glass reactor under nitrogen was charged with concentrated sulfuric acid (200.9 g) and cooled to 0-5°C. Fuming HNO₃ (187.9 g) was carefully added dropwise at a temperature from 0°C to 10°C in 20 min. Then Dichloromethane (2.23 L) was added and the reaction mixture was stirred for 45 min. 6-Hydroxyhexanoic acid potassium salt (110.1g, 1 eq.) was added portion-wise in 30 min at temperature between -5°C to 5°C. The mixture was stirred for 2.5 hours at temperature between -5°C to 5°C and then overnight at 20°C, the reaction was monitored by ¹H-NMR showing 99.6% conversion. Saturated sodium chloride aqueous solution (315.3 g in 1.0 L) was added carefully at a temperature equal to or below 10°C within 25 min. Precipitation of large amount of inorganic salts was observed. The reaction mixture was transferred to a separating funnel taking care of not transferring inorganic salts. The organic layer was decanted, dried over sodium sulfate and concentrated under vacuum (at a temperature equal to or below 40°C) to give crude 6-(nitrooxy)hexanoic acid (105.4 g, 85.2%). HPLC purity = 83.7% with an amount of 6-{[6-(nitrooxy)hexanoyl]oxy}hexanoic acid (compound (IXa) of 8.8%.

Nitration 2: The reaction was performed using 109.97 g of 6-hydroxyhexanoic acid potassium salt to obtain 101.6 g of crude 6-(nitrooxy)hexanoic acid (VIIIa) (82%) that had 89.7% HPLC purity and an amount of 6-{[6-(nitrooxy)hexanoyl]oxy}hexanoic acid (compound (IXa)) of 5.8%.

The two batches were combined and subjected to the following steps without further purification

### Synthesis of 6-(nitrooxy)hexanoyl chloride (Vlllb)

6-(Nitrooxy)hexanoic acid (193 g) was dissolved in dichloromethane (870 mL). The resulting cloudy solution was filtered on glass fibers washed with dichloromethane (130 mL) and the clear solution was analyzed by Karl Fischer (water content = 0.018%). The solution was cooled to 0°C to 5°C under nitrogen. Subsequently, N,N-dimethylformamide (1.2 mL) and oxalyl chloride (141.4 g) were added at 0°C to 5°C within 30 minutes. The reaction mixture was stirred at 0°C to 5°C for 1 hour and 14 hours at 15°C to 20°C. TLC monitoring showed the complete reaction. The mixture was concentrated under vacuum (temperature is equal to or below 40°C) and co-evaporated with dichloromethane (4 x 870 ml) to give 6-(nitrooxy)hexanoyl chloride (212.4 g) with 91.9% yield.

### Preparation of (Z)-7-[(1S,5R,6R,7R)-3-butyl-6-[((E,3S)-3-hydroxy-5-phenyl-pent-1-enyl]-2,4-dioxa-3-borabicyclo[3.2.1)octan-7-yl]-N-ethyl-hept-5-enamide (compound (II))

Methyl tert-butyl ether (3900mL, 14vol.) was charged in a flask. Bimatoprost (249.1 g, 1 eq.) was added and the equipment was rinsed with methyl tert-butyl ether (250 mL, 1 vol.). Butyl boronic acid (70.66 g, 69.2 g in pure material, 1.13 eq.) was added to the resulting suspension in one portion, the equipment was rinsed with methyl tert-butyl ether (250 mL, 1 vol.). The mixture was heated to 40°C for 2 h. The reaction was monitored by ¹H NMR till conversion >97%.

The reaction mixture was cooled to 20°C to 25°C, clarified on a glass filter and washed with methyl tert-butyl ether (250 mL, 1 vol.). The filtrate was concentrated under vacuum at a temperature around 40°C by azeotropic distillation. Rinsing with methyl tert-butyl ether and azeotropic distillation were continued till the water content of (Z)-7-[(1S,5R,6R,7R)-3-butyl-6-[((E,3S)-3-hydroxy-5-phenyl-pent-1-enyl]-2,4-dioxa-3-borabicyclo[3.2.1)octan-7-yl]-N-ethyl-hept-5-enamide (compound (II)) was 0.2%. Compound of formula (II) was analyzed by 1H NMR to determine residual MTBE.

(Z)-7-[(1S,5R,6R,7R)-3-butyl-6-[((E,3S)-3-hydroxy-5-phenyl-pent-1-enyl]-2,4-dioxa-3-borabicyclo[3.2.1)octan-7-yl]-N-ethyl-hept-5-enamide (compound (II)) was obtained in quantitative yield (386.7 g of crude material, uncorrected).

### Preparation of (1S,2E)-3-{(6R,7R)-3-butyl-7[(2Z)-7-(ethylamino)-7-oxohept-2-en-1-yl]-2,4-dioxa-3-borabicyclo[3.2.1]oct-6-yl}-1-(2-phenylethyl)-prop-2-en-1-yl 6-(nitrooxy) hexanoate (XI)

Methyl tert-butyl ether (4100 mL, 11.6 vol.) was charged in a 4L three-neck round-bottomed flask under nitrogen. (Z)-7-[(1S,5R,6R,7R)-3-Butyl-6-[((E,3S)-3-hydroxy-5-phenyl-pent-1-enyl]-2,4-dioxa-3-borabicyclo[3.2.1)octan-7-yl]-N-ethyl-hept-5-enamide (compound (II)) (378 g crude, 1 eq.) was added, equipment was rinsed with methyl tert-butyl ether (510 mL, 1.44 vol.). The resulting solution was cooled to 0°C to 5°C.

4-Dimethylaminopyridine (177.5 g, 2.27 eq.) was added in one portion. A solution of 6-(nitrooxy)hexanoyl chloride (204.4 g, corresponding to 188g in pure, 1.5 eq.) in methyl tert-butyl ether (650 mL, 1.84 vol.) was added dropwise at 0°C to 5°C within 45 min. The dropping funnel was rinsed with methyl tert-butyl ether (40 mL, 0.12 vol.). After stirring for 2 hours at 0°C to 5°C. The mixture was stirred at 15 to 20°C for 17.5 h. HPLC monitoring showed 99.0% conversion. Deionized water (1730 mL, 4.89 vol.) was added within 9 min at a maximum temperature of 25°C.

The mixture was decanted. Aqueous layer was analyzed then discarded. Organic layer was washed with 1M hydrochloric acid aqueous solution. Aqueous layer was analyzed then discarded. Organic layer was washed first with deionized water (1351mL, 5 vol) then with a saturated sodium chloride solution (3x1177 mL, 3x4.25 vol.).

Aqueous layers (pH=4 after the last washing) were analyzed and discarded. Organic layer was dried over sodium sulfate (240 g, 86.8% w/w), washed with methyl tert-butyl ether (616 mL, 2vol.) and concentrated under vacuum to afford (1S,2E)-3-{(6R,7R)-3-butyl-7[(2Z)-7-(ethylamino)-7-oxohept-2-en-1-yl]-2,4-dioxa-3-borabicyclo[3.2.1]oct-6-yl}-1-(2-phenylethyl)-prop-2-en-1-yl 6-(nitrooxy)hexanoate (compound (XI) in quantitative yield (417.4 g, corresponding to 322.1 g of pure product, yield 85.8% pure).

### Synthesis of hexanoic acid, 6-(nitrooxy)-, (1S,2E)-3-[(1R,2R,3S,5R)-2-[(2Z)-7-(ethylamino)-7-oxo-2-hepten-1-yl]-3,5-dihydroxycyclopentyl]-1-(2-phenylethyl)-2-propen-1-yl ester (I) (crude compound).

(1S,2E)-3-{(6R,7R)-3-butyl-7[(2Z)-7-(ethylamino)-7-oxohept-2-en-1-yl]-2,4-dioxa-3-borabicyclo[3.2.1]oct-6-yl}-1-(2-phenylethyl)-prop-2-en-1-yl 6-(nitrooxy) hexanoate (compound (XI)) (407.3 g crude, 1eq.) was dissolved in methanol (3550 mL, 8.9 vol.). The resulting solution was charged in the flask and equipment was rinsed with methanol (1140 mL, 2.8 vol.). The mixture was stirred at 15°C to 25°C for 15 h monitoring the reaction by ¹H-NMR. Methanol was then removed under vacuum at 35°C to 40°C. Methanol (3550 mL, 8.8 vol.) was added and the solution was transferred into a reactor. Reaction was stirred at 20 - 22°C for 16 h. IPC was performed by NMR and showed completion of the reaction. The reaction mixture was concentrated under vacuum at a temperature below 40°C. The residue was dissolved in methyl tert-butyl ether (4350 mL, 10.85 vol.). The resulting solution was washed with deionized water (2190 mL). The aqueous layer (pH=7) was discarded. Organic layer was washed with the sodium chloride solution (2x1930 mL, 2x4.8 vol.). Aqueous layers were discarded. Organic layer was dried over sodium sulfate (395 g, 1 eq w/w), washed with methyl tert-butyl ether (790mL, 2vol.) and concentrated under vacuum at a temperature below 40°C to afford crude hexanoic acid, 6-(nitrooxy)-, (1S,2E)-3-[(1R,2R,3S,SR)-2-[(2Z)-7-(ethylamino)-7-oxo-2-hepten-1-yl]-3,5-dihydroxycyclopentyl]-1-(2-phenylethyl)-2-propen-1-yl ester (compound (I)) (367.1 g, 98.3% yield) having a HPLC purity of 80.4%.

The mixture was purified using silica gel column (750 g x 6, 10.34 vol.) and dichloromethane / methanol as eluent with a gradient 100:0 to 95:5 on a Combiflash. The fractions were monitored by TLC and analyzed by HPLC (%area). Fractions were concentrated under vacuum at temperature equal to or below 50°C to afford 206 g of hexanoic acid, 6-(nitrooxy)-, (1S,2E)-3-[(1R,2R,3S,5R)-2-[(2Z)-7-(ethylamino)-7-oxo-2-hepten-1-yl]-3,5-dihydroxycyclopentyl]-1-(2-phenylethyl)-2-propen-1-yl ester (Compound (I)) that were charged in a 4L three-neck round-bottomed flask and dissolved in absolute ethanol (2100 mL, 10vol.), activated charcoal (21 g, 10% /w) was added and the mixture was stirred at 20°C to 25°C for 0.5 h. The charcoal was filtered and washed with absolute ethanol (210 mL, 1 vol.). The filtrate was concentrated under industrial vacuum at 45°C to 50°C for 4 h then under high vacuum at 45°C to 50°C for 8 h. Monitoring by ¹H NMR in DMSO-d6 showed no residual solvents.

Hexanoic acid, 6-(nitrooxy)-, (1S,2E)-3-[(1R,2R,3S,5R)-2-[(2Z)-7-(ethylamino)-7-oxo-2-hepten-1-yl]-3,5-dihydroxycyclopentyl]-1-(2-phenylethyl)-2-propen-1-yl ester (192.9 g.) was obtained with 62% overall yield from compound (II). HPLC purity was 99.13%.

| Table 1 | | | | | |
|---|---|---|---|---|---|
| Quantitative analysis and chemical yield of 6-(nitrooxy)hexanoic acid prepared according to the process of the present invention (Example 1) and to the process disclosed in WO 2019/162149 (Example 2 - comparative) | | | | | |
| Example | Starting amount of compound (X) (g) | Final amount of Crude Compound (Villa) (g) | HPLC Purity of Compound (Villa) (a/a%) | Amount of compound (IXa) (a/a%) | Effective yield of (Villa) corrected by HPLC data (%) |
| Example 1 | 306.1 | 318.4 | 99.2 | 0.15 | 99.0 |
| Example 2 (Nitration 1) | 110.9 | 105.4 | 83.7 | 8.80 | 76.4 |
| Example 2 (Nitration 2) | 110.0 | 101.6 | 89.7 | 5.82 | 79.6 |

| Table 2 | | | |
|---|---|---|---|
| UHPLC quantitative analysis of compound (I) and its impurity profile prepared according to the process of the present invention (Example 1) and to the process disclosed in WO 2019/162149 (Example 2 - comparative) | | | |
| Example | Content (a/a%) | | |
| | Compound (I) | Impurity (VII) | Impurity (V) |
| 1 | 99.89 | <0.05 | 0.11 |
| 2 | 99.13 | 0.40 | 0.12 |

Table 1 reports the results of the quantitative analysis and chemical yield of 6-(nitrooxy)hexanoic acid (Villa) prepared according to the nitration reactions of the present invention and of the process disclosed in WO 2019/162149. The results show that the addition of water, instead of a saturated solution of NaCl in water (brine) as described in WO 2019/162149, during the work-up of the nitration reaction of the 6-hydroxyhexanoic acid alkali salt led to a crude nitration mixture containing almost pure compound (Villa) (purity of 99.2% (a/a %) and containing a reduced amount of the dimer impurity 6-{ [6-(nitrooxy)hexanoyl]oxy}hexanoic acid (IXa) (0.15% a/a %). Compound (VIIIa) was also obtained in high chemical yield such as 99%. The use of the crude nitration mixture of step 5a) allows to obtain the final product hexanoic acid, 6-(nitrooxy)-, (1S,2E)-3-[(1R,2R,3S,5R)-2-[(2Z)-7-(ethylamino)-7-oxo-2-hepten-1-yl]-3,5-dihydroxycyclopentyl]-1-(2-phenylethyl)-2-propen-1-yl ester (I) that contains and amount of the impurity 6-{[6-(nitrooxy)hexanoyl]oxy}hexanoic acid ester of bimatoprost (VII) less than 0.05%. Indeed, the results of Table 2, which reports the quantitative analysis of compound (I) and of the main impurities prepared according to the process of the invention (Example 1) and to a method discloses in WO 2019/162149 (Example 2 -comparative), show that the process of the invention provides compound (I) with a content of the "dimer impurity" 6-{[6-(nitrooxy)hexanoyl]oxy}hexanoic acid ester of bimatoprost (compound (VII)) below the detection limit of 0.05% and a content of 15-(6-chlorohexanoyl) ester of bimatoprost (compound (V)) of 0.11%. The process disclosed in the prior art leads to compound (I) having a lower chemical purity and a content of compound (VII) from 0.1% to 0.4%.

The results demonstrate that the process of the invention represents an improved process easily transferable to the industrial scale.

## Claims

1. A process for the preparation of hexanoic acid, 6-(nitrooxy)-, (1S,2E)-3-[(1R,2R,3S,5R)-2-[(2Z)-7-(ethylamino)-7-oxo-2-hepten-1-yl]-3,5-dihydroxycyclopentyl]-1-(2-phenylethyl)-2-propen-1-yl ester of formula (I): 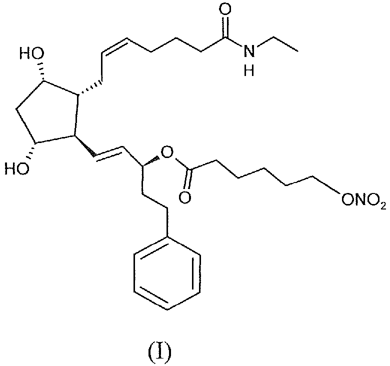 comprising the following steps:
Step A) preparation of the 6-(nitrooxy)hexanoyl chloride (VIIIb) by:
1a) reacting the ε-caprolactone 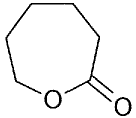 with an inorganic base selected from KOH, NaOH and LiOH in a solvent selected from methanol, ethanol or isopropanol, at a temperature between 20°C and the reflux temperature of the solvent to obtain 6-hydroxyhexanoic acid salt of formula (X) 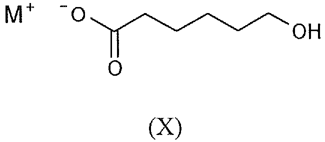 wherein M is K, Na or Li;
2a) purifying the 6-hydroxyhexanoic acid salt of formula (X) obtained in step 1a) which comprises:
i. adding methyl tert-butyl ether to the reaction mixture of step 1a) in a ratio methyl tert-butyl ether / volume of the reaction mixture 2:1;
ii. filtering the solid;
iii. slurring the solid with a mixture of methyl tert-butyl ether, methanol in a ratio of 3:1 to 5:1 and water in an amount of 0.3 - 1 mole of water for mole of ε-caprolactone;
iv. isolating the 6-hydroxyhexanoic acid salt of formula (X) as a solid;
3a) reacting the 6-hydroxyhexanoic acid salt with a mixture of fuming HNO₃ and concentrated H₂SO₄ in dichloromethane, at a temperature ranging from 0°C to 10 °C;
4a) adding water to the nitration mixture of step 3a) while keeping the temperature between 0°C and 5 °C;
5a) separating the organic phase, drying on sodium sulfate said organic phase and distilling off the solvent to obtain crude 6-(nitrooxy)hexanoic acid (Villa) 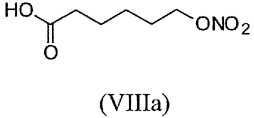
6a) reacting the crude 6-(nitrooxy)hexanoic acid (Villa) with a chlorinating agent in dichloromethane to obtain the crude 6-(nitrooxy)hexanoyl chloride (VIIIb);
Step B) preparation of hexanoic acid, 6-(nitrooxy)-, (1S,2E)-3-[(1R,2R,3S,5R)-2-[(2Z)-7-(ethylamino)-7-oxo-2-hepten-1-yl]-3,5-dihydroxycyclopentyl]-1-(2-phenylethyl)-2-propen-1-yl ester (I) comprising the following steps:
1b) reacting bimatoprost with butyl boronic acid in methyl tert-butyl ether at temperature about 40°C, to obtain compound (II): 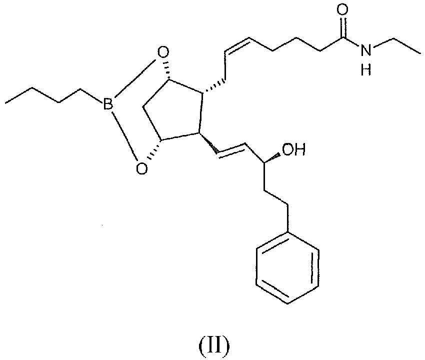
2b) reacting compound (II) with the crude 6-(nitrooxy)hexanoyl chloride (VIIIb) of step 6a) in the presence of 4-dimethylaminopyridine in free form in an aprotic organic solvent, to obtain the compound (XI) 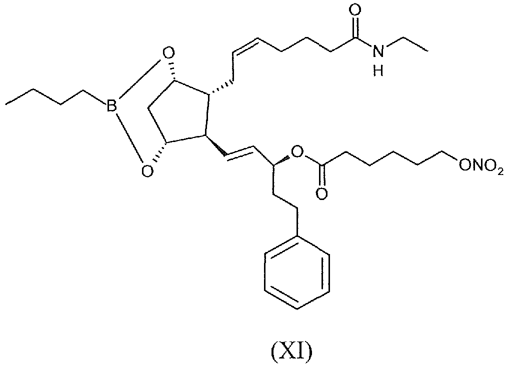
3b) removing the boronate protective group to obtain the crude compound of formula (I);
4b) purifying the crude compound (I) by column chromatography.

2. The process according to claim 1, wherein in step 1a) the inorganic base is KOH and the solvent is methanol and the reaction is carried out at the reflux temperature of methanol

3. The process according to claim 2, wherein in step 3a) the 6-hydroxyhexanoic acid potassium salt is added to a mixture of fuming HNO₃ and concentrated H₂SO₄ in dichloromethane at a temperature from about 0° C to 10°C and the reaction mixture is vigorously stirred for 60 ± 15min at a temperature from 0°C to 5°C.

4. The process according to any of claims 1 to 3, wherein in step 6a) the chlorinating agent is oxalyl chloride.

5. The process according to any of claims 1 to 4, wherein in step 2b) the aprotic solvent is methyl tert-butyl ether at a temperature ranging from 0°C to 20±3°C.

6. The process according to any of claims 1 to 5, wherein in step 2b) the molar ratio of compound (II) to 4-dimethylaminopyridine is from 1:2.0 to 1: 2.4.

7. The process according to any of claims 1 to 6, wherein in step 3b) the solvent used in the reaction is methanol and the reaction is carried out at room temperature.

8. The process according to any of claims 1 to 7, wherein in step 4b) the crude compound (I) is purified using high performance silica gel chromatography and methylene chloride/ methanol as mobile phase.

9. A process for the synthesis of the 6-(nitrooxy)hexanoic acid comprising:
1a) reacting the ε-caprolactone 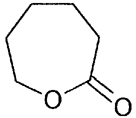 with KOH in a methanol at the reflux temperature of the solvent;
2a)-i. adding methyl tert-butyl ether to the reaction mixture of step 1a) in a ratio methyl tert-butyl ether / volume of the reaction mixture 2:1;
2a)-ii. filtering the solid 6-hydroxyhexanoic acid potassium salt;
2a)-iii. slurring the solid with a mixture of methyl tert-butyl ether, methanol in a ratio of 3:1 to 5:1 and water in an amount of 0.3 - 1 mole of water for mole of ε-caprolactone;
2a)-iv. isolating the 6-hydroxyhexanoic acid potassium salt as a solid;
3a) adding the 6-hydroxyhexanoic acid potassium salt of step 2a) in a mixture of fuming HNO₃ and concentrated H₂SO₄ in dichloromethane, at a temperature ranging from 0°C to 10 °C and vigorously stirring the reaction mixture at a temperature from 0°C to 5°C;
4a) adding water to the nitration mixture of step 3a) while keeping the temperature between 0°C and 5 °C;
5a) separating the organic phase, drying on sodium sulfate said organic phase and distilling off the solvent to obtain 6-(nitrooxy)hexanoic acid **characterized by** a chemical purity equal to or greater than 99% and a content of 6-{[6-(nitrooxy)hexanoyl]oxy}hexanoic acid equal to or less than 0.2%.

## Patentansprüche

1. Verfahren zur Herstellung von Hexansäure-6-(nitrooxy)-(1S,2E)-3-[(1R,2R,3S,5R)-2-[(2Z)-7-(ethylamino)-7-oxo-2-hepten-1 -yl] -3,5 -dihydroxycyclopentyl] -1 -(2-phenylethyl)-2-propen-1 -yl-ester der Formel (I): 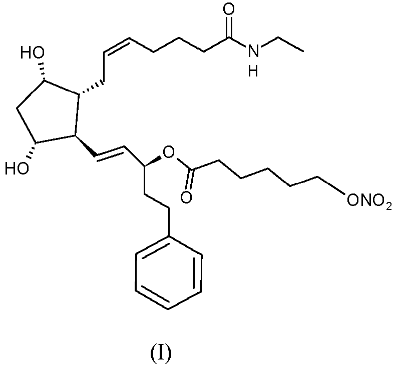 umfassend die folgenden Schritte:
Schritt A) Herstellung von 6-(Nitrooxy)hexanoylchlorid (VIIIb) durch:
1a) Umsetzen des ε-Caprolactons 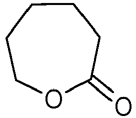 mit einer anorganischen Base, ausgewählt aus KOH, NaOH und LiOH, in einem Lösungsmittel, ausgewählt aus Methanol, Ethanol oder Isopropanol, bei einer Temperatur zwischen 20°C und der Rückflusstemperatur des Lösungsmittels unter Erhalt eines 6-Hydroxyhexansäuresalzes der Formel (X) 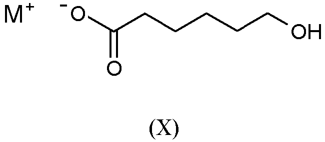 wobei M K, Na oder Li ist;
2a) Reinigen des in Schritt 1a) erhaltenen 6-Hydroxyhexansäuresalzes der Formel (X), umfassend:
i. Zugeben von Methyl-tert.-butylether zum Reaktionsgemisch von Schritt 1a) im Verhältnis Methyl-tert.-butylether/Volumen des Reaktionsgemischs 2:1;
ii. Filtrieren des Feststoffs;
iii. Aufschlämmen des Feststoffs mit einer Mischung aus Methyl-tert.-butylether, Methanol im Verhältnis 3:1 bis 5:1 und Wasser in einer Menge von 0,3 bis 1 Mol Wasser pro Mol ε-Caprolacton;
iv. Isolieren des 6-Hydroxyhexansäuresalzes der Formel (X) als Feststoff;
3a) Umsetzen des 6-Hydroxyhexansäuresalzes mit einer Mischung aus rauchender HNO₃ und konzentrierter H₂SO₄ in Dichlormethan bei einer Temperatur von 0°C bis 10°C;
4a) Zugeben von Wasser zu der Nitriermischung aus Schritt 3a) bei einer Temperatur zwischen 0°C und 5°C;
5a) Abtrennen der organischen Phase, Trocknen der organischen Phase über Natriumsulfat und Abdestillieren des Lösungsmittels unter Erhalt von roher 6-(Nitrooxy)hexansäure (VIIIa) 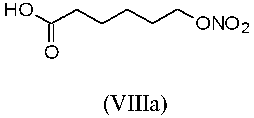
6a) Umsetzen der rohen 6-(Nitrooxy)hexansäure (VIIIa) mit einem Chlorierungsmittel in Dichlormethan unter Erhalt von rohem 6-(Nitrooxy)hexanoylchlorid (VIIIb);
Schritt B) Herstellung von Hexansäure-6-(nitrooxy)-(1S,2E)-3-[(1R,2R,3S,SR)-2-[(2Z)-7-(ethylamino)-7-oxo-2-hepten-1-yl]-3,5-dihydroxycyclopentyl]-1-(2-phenylethyl)-2-propen-1-yl-ester (I), umfassend die folgenden Schritte:
1b) Umsetzen von Bimatoprost mit Butylboronsäure in Methyl-tert.-butylether bei einer Temperatur von etwa 40°C unter Erhalt von Verbindung (II): 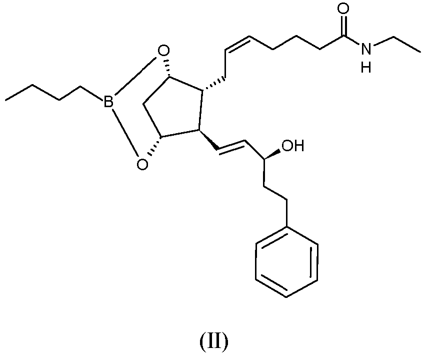
2b) Umsetzen der Verbindung (II) mit dem rohen 6-(Nitrooxy)hexanoylchlorid (VIIIb) aus Schritt 6a) in Gegenwart von 4-Dimethylaminopyridin in freier Form in einem aprotischen organischen Lösungsmittel unter Erhalt der Verbindung (XI) 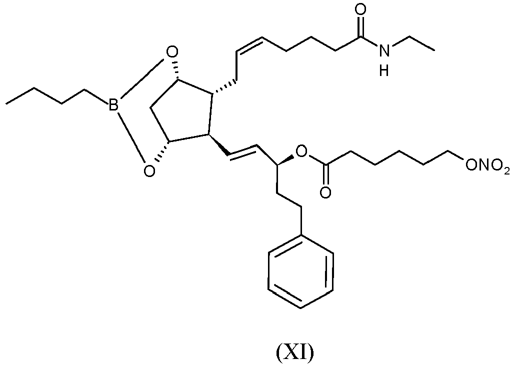
3b) Entfernen der Boronat-Schutzgruppe unter Erhalt der rohen Verbindung der Formel (I);
4b) Reinigen der rohen Verbindung (I) durch Säulenchromatographie.

2. Verfahren nach Anspruch 1, wobei in Schritt 1a) die anorganische Base KOH und das Lösungsmittel Methanol ist und die Reaktion bei der Rückflusstemperatur von Methanol durchgeführt wird

3. Verfahren nach Anspruch 2, wobei in Schritt 3a) das Kaliumsalz der 6-Hydroxyhexansäure zu einem Gemisch aus rauchender HNO₃ und konzentrierter H₂SO₄ in Dichlormethan bei einer Temperatur von etwa 0°C bis 10°C gegeben und das Reaktionsgemisch 60 ± 15 Minuten lang bei einer Temperatur von 0°C bis 5°C kräftig gerührt wird.

4. Verfahren nach einem der Ansprüche 1 bis 3, wobei das Chlorierungsmittel in Schritt 6a) Oxalylchlorid ist.

5. Verfahren nach einem der Ansprüche 1 bis 4, wobei in Schritt 2b) das aprotische Lösungsmittel Methyl-tert.-butylether bei einer Temperatur im Bereich von 0°C bis 20±3°C ist.

6. Verfahren nach einem der Ansprüche 1 bis 5, wobei in Schritt 2b) das molare Verhältnis von Verbindung (II) zu 4-Dimethylaminopyridin 1:2,0 bis 1:2,4 beträgt.

7. Verfahren nach einem der Ansprüche 1 bis 6, wobei in Schritt 3b) das für die Reaktion verwendete Lösungsmittel Methanol ist und die Reaktion bei Raumtemperatur durchgeführt wird.

8. Verfahren nach einem der Ansprüche 1 bis 7, wobei in Schritt 4b) die rohe Verbindung (I) unter Verwendung von Hochleistungs-Kieselgelchromatographie und Methylenchlorid/Methanol als mobile Phase gereinigt wird.

9. Verfahren zur Synthese von 6-(Nitrooxy)hexansäure, umfassend:
1a) Umsetzen des ε-Caprolactons 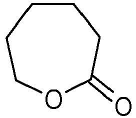 mit KOH in Methanol bei der Rückflusstemperatur des Lösungsmittels;
2a)-i. Zugabe von Methyl-tert.-butylether zum Reaktionsgemisch von Schritt 1a) in einem Verhältnis Methyl-tert.-butylether/Volumen des Reaktionsgemisches 2:1;
2a)-ii. Filtrieren des festen Kaliumsalzes der 6-Hydroxyhexansäure;
2a)-iii. Aufschlämmen des Feststoffs mit einer Mischung aus Methyl-tert.-butylether, Methanol im Verhältnis 3:1 bis 5:1 und Wasser in einer Menge von 0,3 - 1 Mol Wasser pro Mol ε-Caprolacton;
2a)-iv. Isolieren des Kaliumsalzes der 6-Hydroxyhexansäure als Feststoff;
3a) Zugeben des 6-Hydroxyhexansäure-Kaliumsalzes aus Schritt 2a) in einer Mischung aus rauchender HNO₃ und konzentrierter H₂SO₄ in Dichlormethan bei einer Temperatur von 0°C bis 10°C und kräftigem Rühren der Reaktionsmischung bei einer Temperatur von 0°C bis 5°C;
4a) Zugabe von Wasser zu der Nitriermischung aus Schritt 3a) bei einer Temperatur zwischen 0°C und 5°C;
5a) Abtrennen der organischen Phase, Trocknen der organischen Phase über Natriumsulfat und Abdestillieren des Lösungsmittels unter Erhalt von 6-(Nitrooxy)hexansäure, **gekennzeichnet durch** eine chemische Reinheit von 99% oder mehr und einen Gehalt an 6-{[6-(Nitrooxy)hexanoyl]oxy}-hexansäure von 0,2% oder weniger.

## Revendications

1. Procédé pour la préparation de l'ester 6-(nitroxy)-(1S,2E)-3-[(1R,2R,3S,5R)-2-[(2Z)-7-(éthylamino)-7-oxo-2-heptén-1-yl]-3,5-dihydroxy-cyclopentyl]-1-(2-phényléthyl)-2-propén-1-ylique de l'acide hexanoïque, de formule (I) : 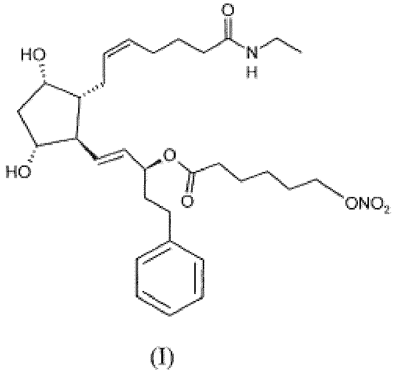 comprenant les étapes suivantes :
étape A) : la préparation du chlorure de 6-(nitroxy)-hexanoyle (Vlllb) par :
1a) la mise en réaction de la ε-caprolactone 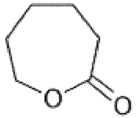 avec une base inorganique choisie parmi le KOH, le NaOH et le LiOH dans un solvant choisi parmi le méthanol, l'éthanol ou l'isopropanol, à une température entre 20 °C et la température de reflux du solvant afin d'obtenir le sel de l'acide 6-hydroxyhexanoïque de formule (X) : 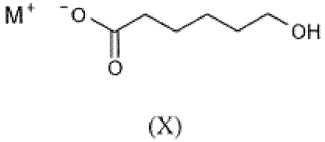 dans laquelle M représente un atome de potassium, un atome de sodium ou un atome de lithium ;
2a) la purification du sel de l'acide 6-hydroxyhexanoïque de formule (X) que l'on a obtenu à l'étape 1a), qui comprend le fait de :
i. ajouter de l'éther méthyl tert-butylique au mélange réactionnel de l'étape 1a) dans un rapport éther méthyl tert-butylique / volume du mélange réactionnel qui s'élève à 2:1 ;
ii. filtrer le solide ;
iii. mettre le solide en suspension avec un mélange d'éther méthyl tert-butylique, de méthanol dans un rapport de 3:1 à 5:1 et d'eau en une quantité de 0,3 à 1 mol d'eau par mole de ε-caprolactone ;
iv. isoler le sel de l'acide 6-hydroxyhexanoïque de formule (X) sous la forme d'un solide ;
3a) la mise en réaction du sel de l'acide 6-hydroxyhexanoïque avec un mélange de HNO₃ fumant et de H₂SO₄ concentré dans du dichlorométhane, à une température se situant dans la plage de 0° C à 10°C;
4a) l'addition d'eau au mélange de nitration de l'étape 3a) tout en maintenant la température entre 0° C et 5° C ;
5a) la séparation de la phase organique, le séchage sur du sulfate de sodium de ladite phase organique et l'élimination du solvant par distillation
afin d'obtenir l'acide 6-(nitroxy)hexanoïque brut (Villa) 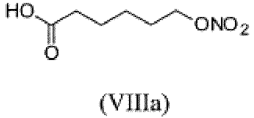
6a) la mise en réaction de l'acide 6-(nitroxy)hexanoïque brut (Villa) avec un agent de chloration dans du dichlorométhane afin d'obtenir le chlorure de 6-(nitroxy)hexanoyle brut (Vlllb) ;
Etape B) : la préparation de l'ester 6-(nitroxy)-(1S,2E)-3-[(1R,2R,3S,5R)-2-[(2Z)-7-(éthylamino)-7-oxo-2-heptén-1-yl]-3,5-dihydroxycyclopentyl]-1-(2-phényléthyl)-2-propén-1-ylique de l'acide hexanoïque (I), comprenant les étapes suivantes :
1b) la mise en réaction de bimatoprost avec de l'acide butyl borique dans de l'éther méthyl tert-butylique à une température d'environ 40 °C afin d'obtenir le composé (II) : 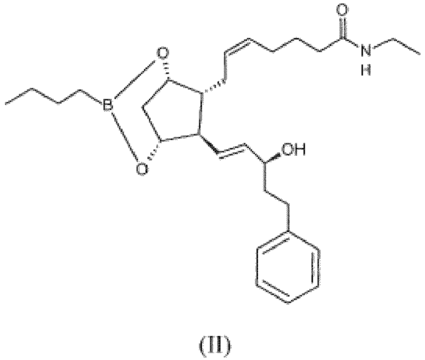
2b) la mise en réaction du composé (II) avec le chlorure de 6-(nitroxy)hexanoyle brut (Vlllb) de l'étape 6a) en présence de la 4-diméthylaminopyridine sous une forme libre dans un solvant organique aprotique, afin d'obtenir le composé (XI) : 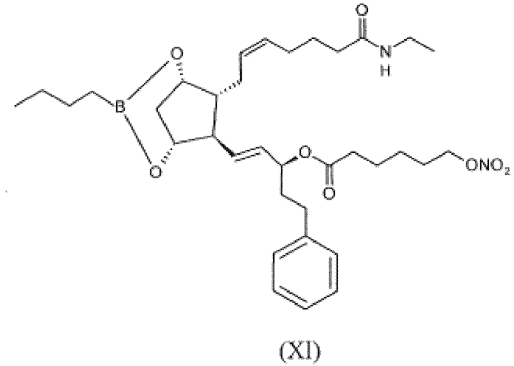
3b) l'élimination du groupe protecteur du borate afin d'obtenir le composé brut de formule (I) ;
4b) la purification du composé brut (I) par colonne chromatographique.

2. Procédé selon la revendication 1, dans lequel, à l'étape 1a), la base inorganique est du KOH et le solvant est du méthanol et la réaction est mise en oeuvre à la température de reflux du méthanol.

3. Procédé selon la revendication 2, dans lequel, à l'étape 3a), on ajoute le sel de potassium de l'acide 6-hydroxyhexanoïque au mélange de HNO₃ fumant et de H₂SO₄ concentré dans du dichlorométhane à une température d'environ 0° C à 10° C, et on agite vigoureusement le mélange réactionnel pendant 60 ± 15 minutes à une température de 0° C à 5° C.

4. Procédé selon l'une quelconque des revendications 1 à 3, dans lequel, à l'étape 6a), l'agent de chloration est le chlorure d'oxalyle.

5. Procédé selon l'une quelconque des revendications 1 à 4, dans lequel, à l'étape 2b), le solvant aprotique est l'éther méthyl tert-butylique à une température se situant dans la plage de 0 °C à 20 ± 3 °C.

6. Procédé selon l'une quelconque des revendications 1 à 5, dans lequel, à l'étape 2b), le rapport molaire du composé (II) à la 4-diméthylaminopyridine s'élève de 1:2,0 à 1:2,4.

7. Procédé selon l'une quelconque des revendications 1 à 6, dans lequel, à l'étape 3b), le solvant utilisé dans la réaction est du méthanol et la réaction est mise en oeuvre à la température ambiante.

8. Procédé selon l'une quelconque des revendications 1 à 7, dans lequel, à l'étape 4b), le composé brut (I) est purifié en utilisant une chromatographie haute performance sur du gel de silice et du chlorure de méthylène / méthanol à titre de phase mobile.

9. Procédé destiné à la synthèse de l'acide 6-(nitroxy)hexanoïque comprenant :
1a) la mise en réaction de la ε-caprolactone 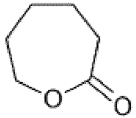 avec du KOH dans du méthanol à la température de reflux du solvant ;
2a)-i. l'addition d'éther méthyl tert-butylique au mélange réactionnel de l'étape 1a) dans un rapport de l'éther méthyl tert-butylique / volume du mélange réactionnel qui s'élève à 2:1;
2a)-ii. la filtration du sel de potassium de l'acide 6-hydroxyhexanoïque solide,
2a)-iii. la mise en suspension du solide avec un mélange d'éther méthyl tert-butylique, de méthanol dans un rapport de 3:1 à 5:1 et d'eau en une quantité de 0,3 - 1 mole d'eau par mole de la ε-caprolactone ;
2a)-iv. l'isolation du sel de potassium de l'acide 6-hydroxyhexanoïque sous la forme d'un solide ;
3a) l'addition du sel de potassium de l'acide 6-hydroxyhexanoïque de l'étape 2a) dans un mélange de HNO₃ fumant et de H₂SO₄ concentré dans du dichlorométhane, à une température se situant dans la plage de 0° C à 10° C et l'agitation vigoureuse du mélange réactionnel à une température de 0° C à 5° C ;
4a) l'addition d'eau au mélange de nitration de l'étape 3a) tout en maintenant la température entre 0° C et 5° C ;
5a) la séparation de la phase organique, le séchage de ladite phase organique sur du sulfate de sodium et l'élimination du solvant par distillation afin d'obtenir l'acide 6-(nitroxy)hexanoïque **caractérisé par** une pureté chimique égale ou supérieure à 99 % et par une teneur en acide 6-{[6-(nitroxy)hexanoyl]oxy}hexanoïque égale ou inférieure à 0,2 %.
